# EUROPÄISCHE PATENTANMELDUNG

(11) **EP 2 172 151 A1**
(43) Veröffentlichungstag der Anmeldung: **07.04.2010**
(21) Anmeldenummer: 08017377.6
(22) Anmeldetag: 02.10.2008
(51) Int. Cl.: A61B 5/00, G06F 19/00

(54) **Medizinisches Patientengerät**

(71) Anmelder: F.HOFFMANN-LA ROCHE AG, 4070 Basel (CH); Roche Diagnostics GmbH, 68305 Mannheim (DE)
(72) Erfinder: Kraemer, Uwe, 68549 Ilvesheim (DE); Liedtke, Sebastian, 69717 Heidelberg (DE)
(74) Vertreter: Hössle, Markus

(57) **Zusammenfassung**

Medizinisches Patientengerät (10) mit einer medizinischen Messeinheit zur Detektion und Verarbeitung von analysespezifischen Signalen, einer Recheneinheit (12), einer Benutzerschnittstelle (16), einer Konfigurationsdatenschnittstelle (24) zum Empfang von Konfigurationsdateien und mit einer Speichereinheit (14), wobei das medizinische Patientengerät (10) derart ausgestaltet und eingerichtet ist, dass ein Nutzer mittels der Benutzerschnittstelle (16) Konfigurationsdateien über die Konfigurationsdatenschnittstelle (24) in das Patientengerät (10) laden und in der Speichereinheit (14) speichern und unter Zugriff auf die heruntergeladenen und in der Speichereinheit (14) gespeicherten Konfigurationsdateien eine Konfiguration der Benutzerschnittstelle (16) vornehmen kann.

## Beschreibung

### Technisches Gebiet

Die vorliegende Erfindung betrifft ein medizinisches Patientengerät und insbesondere ein medizinisches Patientengerät zur Durchführung und/oder Analyse medizinischer Selbsttest und/oder zur Selbstverabreichung von Medikamenten oder dergleichen jeweils durch den Patienten selbst.

### Beschreibung des Standes der Technik

Im medizinischen Bereich ist es bekannt, tragbare Patientengeräte zum Sammeln von Patientendaten einzusetzen. Häufig sind diese tragbaren Geräte mit zentralen Datenverarbeitungsgeräten verbunden, in denen eine Überwachung, Auswahl, Analyse usw. der Daten entweder von medizinischem Personal, Ärzten oder auch automatisiert vorgenommen wird. Derartige medizinische Patientengeräte sind beispielsweise als Blutzuckermessgeräte bekannt und auf dem Markt erhältlich und werden eingesetzt, um Blutzuckerwerte von Diabetikern zu sammeln und zu überwachen. Aus der EP 1 559 364 A1 ist bspw. ein drahtloses Diabetesüberwachungssystem bekannt, bei dem dem Patienten nach Übermittlung seiner Blutzuckerwerte an eine Zentrale geeignete Verhaltensanweisungen über ein mobiles Telefon mitgeteilt werden. Ein weiteres System ist aus der US 2005/0089150 A1 bekannt, bei dem über Telefon und tragbare Geräte eine interaktive Anweisung mittels Spracherkennungssystemen und softwareerzeugten Anweisungen an den Nutzer/Patienten erfolgt.

An Diabetes Mellitus erkrankte Personen müssen bestrebt sein, ihren Blutzuckerwert stets in einem bestimmten Bereich zu halten. Wird der angestrebte Bereich überschritten, muss Insulin gespritzt werden. Wird der angestrebte Bereich unterschritten, muss z.B. Zucker oral (über die Nahrung oder ein Getränk) zugeführt werden. Wird der angestrebte Bereich über eine längere Zeit überschritten, besteht die Gefahr von ernsthaften gesundheitlichen Komplikationen, wie Blindheit, Nierenschädigung, Absterben von Gliedmaßen oder Neuropathie. U.a. bei kurzfristigem, starkem Überschreiten des Bereiches kann es zu Übelkeit, Schwindel, Schweißausbrüchen und sogar zu Zuständen der Verwirrtheit kommen. Bei einem kurzzeitigen Unterschreiten des angestrebten Bereichs kann es ebenfalls zu Übelkeit, Schwindel, Schweißausbrüchen, Verwirrtheit und - im schlimmsten Falle - zum Tode des Diabetikers kommen. Daher ist es dringend geboten, dass ein Diabetiker jederzeit seinen Blutzuckerstatus kennt und ggf. eigenständig geeignete Maßnahmen einleiten kann, um ein Über- oder Unterschreiten des Blutzuckerwerts aus dem angestrebten Bereich zu vermeiden. Hierzu werden bereits seit geraumer Zeit Blutzuckermessgeräte verwendet, wie sie bspw. aus der DE 10 2004 057 503 A1 bekannt sind und von der Anmelderin unter der eingetragenen Marke Accu-Chek^{®} vertrieben werden. Im Idealfall handhabt der Diabetiker die Messung des Blutzuckerwerts und sich daraus ergebende Maßnahmen in Eigenverantwortung. Der Blutzuckerwert unterliegt starken Schwankungen in Abhängigkeit von den Insulingaben (in der Regel werden unterschiedlich wirkende Insuline gleichzeitig verwendet), von den zugeführten Zuckermengen und anderen physiologisch auf den Zuckerstoffwechsel wirkenden Nahrungs- und Genussmitteln. Ebenfalls auf den Zuckerstoffwechsel wirken körperliche Bewegung, Stress, Krankheit u.v.a.m. Da nicht jeder Organismus in gleicher Weise auf diese physiologischen Größen reagiert, muss jeder Diabetiker seine eigenen physiologischen Reaktionen kennenlernen. Hierzu ist es unerlässlich, dass der Patient sein Patientengerät regelmäßig benutzt und einsetzt.

Aus der US 2003/0050537 A1 ist ein Gerät zur interaktiven Belohnung bekannt, wobei das Gerät eine Belohnung ausgibt, wenn ein medizinischer Test von einem Patienten durchgeführt wurde oder der Patient die Resultate seiner medizinischer Test auf einem gewünschten Niveau gehalten hat. Das Gerät besteht beispielsweise aus einem Blutzuckermessgerät, das mit einem unabhängigen weiteren Gerät verbunden werden kann, das wiederum zur Motivierung bzw. Belohnung des Patienten dient. Bei diesem weiteren unabhängigen Gerät handelt es sich insbesondere um eine Videospielkonsole oder ein Mobiltelefon.

### Zusammenfassung der Erfindung

Demgegenüber wird erfindungsgemäß ein medizinisches Patientengerät mit den Merkmalen des Anspruchs 1 vorgeschlagen.

Ein medizinisches Patientengerät, wie beispielsweise ein Blutzuckermessgerät, umfasst ein Steuermodul mit einem Mikroprozessor, einen mit dem Mikroprozessor verbundenen Speicher, ein medizinisches Modul (Messeinheit) zur Detektion und Verarbeitung analysespezifischer Daten mit einer Schnittstelle zur Aufnahme bzw. Abgabe medizinisch relevanter Daten sowie eine Benutzerschnittstelle, über die Ergebnisse medizinischer Test ausgegeben werden können sowie ein Benutzer das medizinische Patientengerät beispielsweise in an sich bekannter Art und Weise steuern und bedienen kann.

Des weiteren umfasst das medizinische Patientengerät ein Konfigurationsmodul, das mit dem Mikroprozessor verbunden und mittels der Benutzerschnittstelle durch einen Benutzer bedienbar ist und das zu einer benutzerseitigen Konfiguration des Blutzuckermessgerätes und insbesondere der Benutzerschnittstelle ausgelegt ist. Dadurch wird einem Benutzer ermöglicht, sein medizinisches Patientengerät an seine Bedürfnisse bzw. Vorlieben anzupassen und zu individualisieren. Hierzu greift das Konfigurationsmodul auf über eine entsprechend ausgestaltete Schnittstelle des Patientengeräts von externen Quellen geladene Daten zu. Die Konfigurationsmöglichkeiten können dabei direkte Konfigurationseinstellungen wie beispielsweise graphische Konfigurationsdaten wie beispielsweise Fotos, Ikonen usw. und/oder Tonkonfigurationsdateien wie akustische Signale umfassen. Der Nutzer kann somit Konfigurationsdaten über die Konfigurationsdatenschnittstelle von einer externen Quelle in das Patientengerät laden.

Dadurch wird dem Benutzer eines medizinischen Patientengerätes wie beispielsweise einem Blutzuckermessgerät ermöglicht, das Patientengerät an seine Bedürfnisse anzupassen und zu individualisieren, was mit einer erhöhten Identifikation des Benutzers mit seinem Patientengerät verbunden ist. Patienten, wie beispielsweise Diabetiker, sind gezwungen, ein Patientengerät regelmäßig, im Zweifel mehrmals täglich, zu verwenden. Von der regelmäßigen Verwendung hängt im Ernstfall das weitere Wohlergehen des Patienten ab. Allerdings wurde beobachtet, dass eine nicht zu vernachlässigende Zahl von Patienten sich nur schwer mit ihrem Patientengerät anfreunden können und beispielsweise Abneigungen gegen voreingestellte Alarmtöne, andere akustische Signale oder auch graphische Darstellungen auf der Anzeigeeinheit der Benutzerschnittstelle entwickeln. Mit der erfindungsgemäßen Ausgestaltung eines medizinischen Patientengerätes wird jedem Patienten die Möglichkeit gegeben, die technischen Parameter seines Patientengeräts an seine persönlichen Bedürfnisse anzupassen. Durch die damit einhergehende Individualisierung des Geräts wird ein deutlich höherer Identifikationsgrad des Patienten mit seinem Patientengerät erreicht, was wiederum mit einer freudigeren Benutzung des Geräts einhergeht.

Die Erfindung umfasst auch ein Patientensystem mit einem erfindungsgemäßen medizinischen Patientengerät und einer Konfigurationsdateien enthaltenden Datenbank, wobei eine Verbindung zwischen dem medizinischen Patientengerät und der Datenbank über die Konfigurationsdatenschnittstelle des medizinischen Patientengeräts aufgebaut wird, derart dass eine oder mehrere Konfigurationsdateien von der Datenbank in das medizinische Patientengerät geladen und dort gespeichert werden können. Die Verbindung mit der Datenbank kann dabei nach bekannten Methoden, bspw. mittels Kabel, Funk, Internet, Infrarot, Bluetooth, WLAN, o.dgl. hergestellt werden. Die Datenbank kann jede Art externer Festplatte sein, bspw. in einem Server, der über Internet, ein Intrant, WLAN usw. angebunden ist, oder bspw. in er tragbaren Gerät, wie einem digitalen Aufnahmegerät für Standbilder, bewegte Bilder und/oder Ton, oder bspw. in einem tragbaren Computer usw.

Die vorliegende Beschreibung deckt auch ein Computerprogramm mit Programmcode ab, der eine Umsetzung der Erfindung bewirkt, wenn das Computerprogramm auf einer Recheneinheit eines medizinischen Patientengeräts abläuft. Das Computerprogramm wird sowohl als Computerprogramm selbst als auch auf einem computerlesbaren Medium gespeichert beansprucht.

Weitere Vorteile und Ausgestaltungen der Erfindung ergeben sich aus der Beschreibung und der beiliegenden Zeichnung.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispieles in der Zeichnung schematisch dargestellt und wird im folgenden unter Bezugnahme auf die Zeichnung ausführlich beschrieben.

### Kurzbeschreibung der Zeichnung

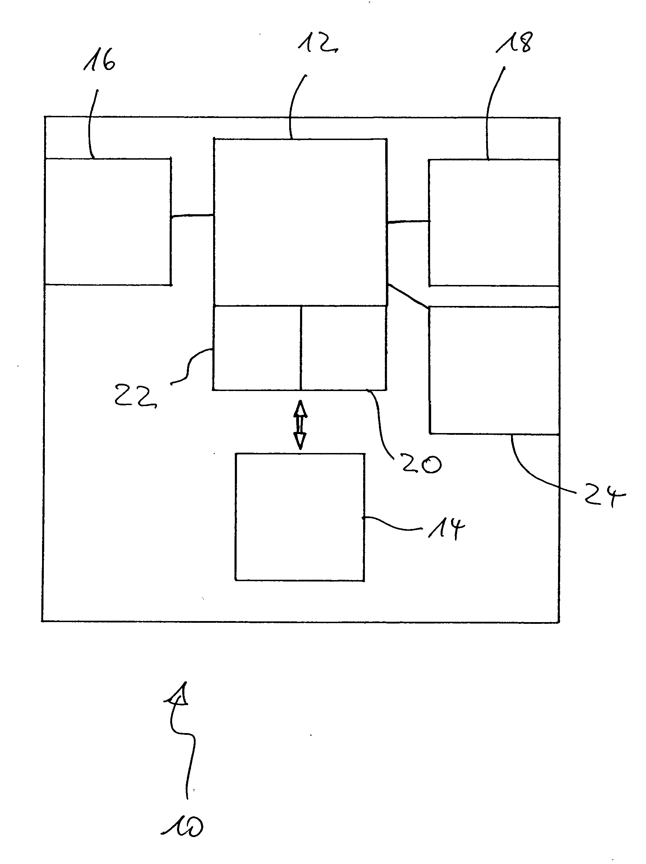

Figur 1 zeigt eine Blockbilddarstellung eines erfindungsgemäßen medizinischen Patientengeräts.

### Ausführliche Beschreibung

Die einzige Figur zeigt eine Blockbilddarstellung eines erfindungsgemäßen medizinischen Patientengeräts 10. Das medizinische Patientengerät 10 umfasst eine Recheneinheit 12, bei der es sich um einen geeigneten Mikroprozessor handeln kann. Die Recheneinheit 12 ist mit einer Speichereinheit 14 verbunden, die zur Speicherung von für den Betrieb des medizinischen Patientengeräts 10 benötigten Daten dient. Des weiteren umfasst das medizinische Patientengerät 10 eine Messeinheit 18 zum Erheben von medizinischen Messdaten (wie bspw. Blutzuckerwerte) zur Verarbeitung in dem Patientengerät. Die Messeinheit verfügt über ein analytisches Testelement, also eine zur Erhebung analysespezifischer Daten ausgebildete Schnittstelle. Die Analyse der Daten erfolgt in an sich bekannter Weise, bspw. auf elektrochemischem oder detektionsphotometrischem Wege.

Diese medizinischen Messdatensätze werden von der Recheneinheit 12 in einem hierfür vorgesehenen Messdatenmodul 20 verarbeitet und dem Nutzer/Patienten über eine hierfür vorgesehene Benutzerschnittstelle 16 in geeigneter Weise optisch/grafisch und ggf. akustisch ausgegeben. So werden beispielsweise Messdatenergebnisse und daraus errechnete weitere relevante Daten dem Nutzer über eine Anzeigeeinheit (nicht dargestellt) als Zahlenwerte und/oder Grafik dargestellt. Die Messwerte können für sehbehinderte Patienten auch akustisch ausgegeben werden. Des weiteren können Grenzwertüberschreitungen, nicht eingehaltene Zeiten usw. durch Signaltöne angezeigt werden.

Die für den Betrieb des medizinischen Patientengeräts 10 benötigten Daten können auf unterschiedliche Weise in der Speichereinheit 14 abgespeichert werden. Zum einen können Daten bereits herstellerseitig vorab gespeichert werden. Zum anderen können Daten von einem Nutzer über die Benutzerschnittstelle 16 eingegeben werden. Schließlich können Daten über weitere hierfür vorgesehene Schnittstellen in das medizinische Patientengerät 10 eingegeben werden. Bei diesen Schnittstellen handelt es sich beispielsweise um eine in die Messeinheit 18 integrierte Medizindatenschnittstelle und eine Konfigurationsdatenschnittstelle 24.

Die Medizindatenschnittstelle dient bspw. dazu, von dem Messdatenmodul 20 errechnete Werte an andere angeschlossene bzw. zugeordnete Geräte auszugeben. Es handelt sich somit um eine kombinierte Eingangs-/Ausgangsschnittstelle, die selbstverständlich auch als zwei Schnittstellen ausgeführt sein kann.

Die Konfigurationsdatenschnittstelle 24 dient dazu, konfigurationsrelevante Datensätze in das medizinische Patientengerät 10 zu laden und dort in der Speichereinheit 14 zu speichern. Bei diesen konfigurationsrelevanten Datensätzen (oder kurz Konfigurationsdateien) handelt es sich um Daten bzw. Datensätze bzw. Dateien, mit deren Hilfe ein Nutzer des medizinischen Patientengeräts 10 eine Konfiguration der grafischen Benutzerschnittstelle 16 vornehmen kann. Beispielsweise handelt es sich um Bilddaten bzw. Bilddateien, die der Nutzer zur Anzeige in der Anzeigeeinheit auswählen kann (wie z.B. Hintergrundbilder/-fotos oder auch Ikonen, mit deren Hilfe bestimmte Routinen der Recheneinheit und/oder Funktionen des medizinischen Patientengeräts aufgerufen werden können). Hierfür verfügt das Patientengerät über einen (nicht dargestellten) geeigneten Grafikprozessor. Beispielsweise handelt es sich auch um Tondateien, die der Nutzer auswählen kann und die, bestimmten Ereignissen zugeordnet, durch die Benutzerschnittstelle akustisch wiedergegeben werden, wie z.B. als Alarmsignal bei Überschreiten oder Unterschreiten eines medizinischen Grenzwertes. Bei dem Tondateien kann es sich um Töne, Musikaufnahmen, Melodien und/oder gesprochenen oder gesungenen Text, wie bspw. Ansagen, handeln. Hierfür verfügt das Patientengerät über einen geeigneten (nicht dargestellten) Soundprozessor mit zugeordnetem Lautsprecher.

Des weiteren können auch bewegte Bilder, wie bspw. Filme, Bildschirmschoner, Animationen, usw., zur optischen und ggf. auch akustischen Konfiguration der Benutzerschnittstelle über die Konfigurationsdatenschnittstelle in das Patientengerät geladen und in der Speichereinheit gespeichert werden, damit der Patient Konfigurationen unter Verwendung dieser Dateien vornehmen kann.

Die Einrichtung der benutzereigenen Konfiguration kann erfindungsgemäß derart erfolgen, dass das Aktivieren (also das Anzeigen und/oder Abspielen) einer von dem Nutzer ausgewählten Datei von einem zugeordneten Ereignis ausgelöst wird. Das zugeordnete Ereignis kann bspw. ein Messwert bzw. ein Messwertintervall sein, d.h. wenn ein erhobener Messwert einen vorbestimmten Wert hat oder in einem vorbestimmten Intervall liegt, dann wird die zugeordnete Datei aktiviert (dabei kann es sich um Intervalle von einem ersten Messwert bis zu einem zweiten Messwert handeln, oder um offene Intervalle, die alle Werte ober- oder unterhalb eines bestimmten Messwertes abdecken, oder um konkrete Messwerte; die Messwerte oder Messwertintervalle können aus einer Liste ausgewählt oder individuell vorgegeben werden).

Dies gestattet es dem Nutzer, positiven Ereignissen, d.h. für ihn günstigen Messwerten, eine Datei mit einem für ihn positiven Inhalt, bspw. eine Lieblingsmelodie, einen Fanfarenstoß, ein Lieblingsbild, einen Lieblingsclip, eine Lieblingsfarbe, usw., zuzuordnen, was eine Belohnung bzw. Motivation für ein Verhalten darstellt, das zu einem "guten" Messwert führt. Dies ist insbesondere für Kinder oder ältere Personen vorteilhaft, deren Geräte von einem Arzt oder einer betreuenden Person entsprechend eingestellt werden könnten. Dies eröffnet darüber hinaus auch die Möglichkeit, eine Messwertwiedergabe vollständig durch Bilder und/oder Töne/Melodien zu gestalten, was ebenfalls für Kinder und ältere Personen die Benutzung eines Patientengerätes stark vereinfacht bzw. überhaupt erst ermöglicht, da so ein vereinfachtes Verständnis der Messwertangaben erzeugt wird (so kann bspw. bspw. guten Messwertintervallen eine Datei mit der Farbe Grün zugeordnet werden, weniger guten, aber akzeptablen Messwertbereichen die Farbe Gelb/Orange und kritischen Messwertbereichen die Farbe Rot (Ampelprinzip). Darüber hinaus können selbstverständlich auch übliche Systemeinstellungen, wie bspw. Wecker- oder Alarm/Erinnerungsfunktionen, entsprechend benutzerseitig gestaltet und konfiguriert werden.

Die Konfigurationsdaten können wie beschrieben über die Konfigurationsdatenschnittstelle 24 in das medizinische Patientengerät 10 geladen werden. Weitere Konfigurationsmöglichkeiten umfassen Einstellung der Schriftart und Schriftgröße, was insbesondere bei Blutzuckermessgeräten für eine einwandfreie Bedienung wichtig ist, da viele Diabetiker Sehbeinträchtigungen unterliegen.

Weitere Konfigurationsmöglichkeiten umfassen Geräusche und Töne des Patientengeräts sowie Leuchtsignale der Anzeigeeinheit. Auch Vibrationseinstellungen sind möglich.

Für die Durchführung der Konfiguration durch den Benutzer kann die Recheneinheit 12 ein Konfigurationsmodul 22 umfassen. Das Konfigurationsmodul 22 ist derart programmiert, dass der Nutzer über eine Benutzeroberfläche die Auswahl der in der Speichereinheit zur Verfügung stehenden Konfigurationsdateien angezeigt bekommt, Er kann dann eine Auswahl treffen, und diese Auswahl einem bestimmten Ereignis oder einer bestimmten Aufgabe oder einer bestimmten Anzeige usw. zuordnen. Falls er neue Konfigurationsdateien in das Patientengerät laden möchte, kann er dies ebenfalls durch Unterstützung des Konfigurationsmoduls tun. Das Konfigurationsmodul stellt somit das Betriebsprogramm zur Verwaltung und Einstellung der Konfigurationsdateien dar.

Der Begriff des medizinischen Patientengeräts ist nicht auf das beschriebene Blutzuckermessgerät beschränkt, sondern erstreckt sich auf alle Geräte, die medizinische Abläufe betreffen und von einem Patienten selbst verwendet bzw. eingesetzt werden. Dies können insbesondere auch Blutdruckmessgeräte, Pumpen, Kontrolleinheiten, Verabreichungssysteme, Stechhilfen usw. sein. So kann bei Stechhilfen beispielsweise eine Konfigurationsmöglichkeit darin bestehen, synchron mit dem Stich eine vom Patienten ausgewählte Tondatei abzuspielen, wodurch der Patient vom Stich abgelenkt wird und ggf. weniger Schmerz wahrnimmt. Dies kann eine Verwendung des Geräts für Kinder und Jugendliche, die inzwischen einen hohen Anteil an Diabetikern stellen, vereinfachen. Die Erfindung ermöglicht somit eine Personalifizierung eines medizinischen Patientengeräts, wodurch es dem Patienten ermöglicht wird, eine größere Nähe zu seinem Gerät aufzubauen und es besser in seinen Alltagsablauf zu integrieren.

## Patentansprüche

1. Medizinisches Patientengerät (10) mit einer medizinischen Messeinheit zur Detektion und Verarbeitung von analysespezifischen Signalen, einer Recheneinheit (12), einer Benutzerschnittstelle (16), einer Konfigurationsdatenschnittstelle (24) zum Empfang von Konfigurationsdateien und mit einer Speichereinheit (14), wobei das medizinische Patientengerät (10) derart ausgestaltet und eingerichtet ist, dass ein Nutzer mittels der Benutzerschnittstelle (16) Konfigurationsdateien über die Konfigurationsdatenschnittstelle (24) in das Patientengerät (10) laden und in der Speichereinheit (14) speichern und unter Zugriff auf die heruntergeladenen und in der Speichereinheit (14) gespeicherten Konfigurationsdateien eine Konfiguration der Benutzerschnittstelle (16) vornehmen kann.

2. Medizinisches Patientengerät (10) nach Anspruch 1, bei dem die Konfigurationsdatenschnittstelle (24) eine drahtlose Schnittstelle ist.

3. Medizinisches Patientengerät (10) nach Anspruch 1 oder 2, bei dem die Konfiguration der Benutzerschnittstelle (16) eine grafische/optische Konfiguration einer Anzeigeeinheit der Benutzerschnittstelle (16) umfasst.

4. Medizinisches Patientengerät (10) nach einem der Ansprüche 1 bis 3, bei dem die Konfiguration der Benutzerschnittstelle (16) eine akustische Konfiguration einer Lautsprechereinheit der Benutzerschnittstelle (16) umfasst.

5. Medizinisches Patientengerät (10) nach einem der Ansprüche 1 bis 4, bei dem die Konfiguration durch Zuordnung einer Konfigurationsdatei zu einem Messwertintervall erfolgt.

6. Medizinisches Patientengerät (10) nach einem der Ansprüche 1 bis 5, bei dem die Konfiguration mittels Dateien erfolgt, die bewegte Bilder enthalten.

7. Computerprogramm zur Steuerung eines medizinischen Patientengeräts (10), wobei das Computerprogramm Programmcode umfasst, der bewirkt, dass ein Nutzer mittels einer Benutzerschnittstelle (16) des medizinischen Patientengeräts (10) Konfigurationsdateien über die Konfigurationsdatenschnittstelle (24) in das Patientengerät (10) laden und in einer Speichereinheit (14) speichern und unter Zugriff auf die heruntergeladenen und in der Speichereinheit (14) gespeicherten Konfigurationsdateien eine Konfiguration der Benutzerschnittstelle (16) vornehmen kann, wenn das Computerprogramm auf einer Recheneinheit (12) des medizinischen Patientengeräts (10) abläuft.

8. Computerprogramm nach Anspruch 6, das auf einem computerlesbaren Medium gespeichert ist.

9. Patientensystem mit einem medizinischen Patientengerät (10) nach einem der Ansprüche 1 bis 6 und einer Konfigurationsdateien enthaltenden Datenbank, wobei eine Verbindung zwischen dem medizinischen Patientengerät (10) und der Datenbank über die Konfigurationsdatenschnittstelle (24) des medizinischen Patientengeräts (10) aufgebaut wird, derart dass eine oder mehrere Konfigurationsdateien von der Datenbank in das medizinische Patientengerät (10) geladen und dort gespeichert werden können.

10. Patientensystem nach Anspruch 9, bei dem die Datenbank auf einer Festplatte eines tragbaren Aufnahmegeräts ist.

11. Patientensystem nach Anspruch 9, bei dem die Datenbank auf einem stationären Festplattenspeicher ist.

12. Patientensystem nach Anspruch 9, bei dem die Datenbank über ein Datennetz anbindbar ist.

13. Patientensystem nach Anspruch 12, bei dem das Datennetz ein LAN, ein Intranet, das Internet o.dgl. ist.
